(19) Europäisches Patentamt
European Patent Office
Office européen des brevets

(11) **EP 2 839 864 A1**

(12) **DEMANDE DE BREVET EUROPEEN**

(43) Date de publication:
**25.02.2015 Bulletin 2015/09**

(51) Int Cl.:
**A63B 69/38** (2006.01)  **G09B 19/00** (2006.01)

(21) Numéro de dépôt: **14181478.0**

(22) Date de dépôt: **19.08.2014**

(84) Etats contractants désignés:
**AL AT BE BG CH CY CZ DE DK EE ES FI FR GB GR HR HU IE IS IT LI LT LU LV MC MK MT NL NO PL PT RO RS SE SI SK SM TR**
Etats d'extension désignés:
**BA ME**

(30) Priorité: **20.08.2013 FR 1358082**

(71) Demandeur: **BABOLAT VS**
**69007 Lyon (FR)**

(72) Inventeurs:
• **Mace, Pierre**
  **69007 Lyon (FR)**
• **Gauthier, Fabien**
  **69007 Lyon (FR)**

(74) Mandataire: **Myon, Gérard Jean-Pierre et al**
**Cabinet Lavoix**
**62, rue de Bonnel**
**69003 Lyon (FR)**

(54) **Méthode de calcul d'un paramètre représentatif de l'endurance d'un joueur de tennis**

(57)     Cette méthode de calcul d'un paramètre (P) représentatif de l'endurance d'un joueur de tennis au cours d'une période de jeu comprenant une série de sessions de jeu, caractérisée en ce qu'elle comprend au moins des étapes consistant à : a) mesurer, à l'aide d'un système de mesure comprenant des capteurs implantés sur une raquette (1) utilisée par le joueur, l'accélération linéaire et la vitesse angulaire de la raquette (1) lors de chaque coup frappé par le joueur au cours d'une session de jeu, b) calculer (100), au moyen d'une unité de calcul, la puissance (W) développée par le joueur lors de chaque coup à partir des accélérations linéaires et des vitesses angulaires mesurées à l'étape a), calculer (101) au moyen de l'unité de calcul, la puissance moyenne (Wm) développée par le joueur pendant la dernière session de jeu, la puissance moyenne étant définie comme la moyenne des puissances (W) développées lors de chaque coup par le joueur calculées à l'étape b), d) calculer (102) au moyen de l'unité de calcul l'énergie (E) dépensée par le joueur pendant la dernière session de jeu, l'énergie dépensée étant égale au nombre (n) de coups exécutés par le joueur au cours de la dernière session de jeu multipliée par la puissance moyenne (Wm) calculée à l'étape c) élevée à un exposant (i) prédéterminé, e) calculer (103) au moyen de l'unité de calcul la valeur du paramètre (P), cette valeur étant définie par le produit du nombre (N) de sessions de jeu réalisées par semaine au cours de la période de jeu, multiplié par l'énergie (E) calculée à l'étape d).

FIG.2

**Description**

L'invention concerne une méthode de calcul d'un paramètre représentatif de l'endurance d'un joueur de tennis au cours d'une session de jeu.

Pour la pratique du tennis, il est avantageux de pouvoir observer les performances, notamment physiques, d'un joueur. Il est notamment intéressant de connaître l'évolution de l'endurance en fonction de l'intensité et de la fréquence de jeu, afin de permettre au joueur d'améliorer ses capacités physiques.

A cet effet, l'invention concerne une méthode de calcul d'un paramètre représentatif de l'endurance d'un joueur de tennis au cours d'une période de jeu comprenant une série de sessions de jeu. Cette méthode est caractérisée en ce qu'elle comprend au moins des étapes consistant à:

- a) mesurer, à l'aide d'un système de mesure comprenant des capteurs implantés sur une raquette utilisée par le joueur, l'accélération linéaire et la vitesse angulaire de la raquette lors de chaque coup frappé par le joueur au cours d'une session de jeu ;
- b) calculer, au moyen d'une unité de calcul, la puissance développée par le joueur lors de chaque coup à partir des accélérations linéaires et des vitesses angulaires mesurées à l'étape a) ;
- c) calculer au moyen de l'unité de calcul, la puissance moyenne développée par le joueur pendant la dernière session de jeu, la puissance moyenne étant définie comme la moyenne des puissances développées lors de chaque coup par le joueur calculées à l'étape b) ;
- d) calculer au moyen de l'unité de calcul l'énergie dépensée par le joueur pendant la dernière session de jeu, l'énergie dépensée étant égale au nombre de coups exécutés par le joueur au cours de la dernière session de jeu multipliée par la puissance moyenne calculée à l'étape c) élevée à un exposant prédéterminé ;
- e) calculer au moyen de l'unité de calcul la valeur du paramètre, cette valeur étant définie par le produit du nombre de sessions de jeu réalisées par semaine au cours de la période de jeu, multiplié par l'énergie calculée à l'étape d).

Grâce à l'invention, un joueur peut évaluer sa capacité à enchaîner les sessions de jeu à haute intensité de frappe dans un intervalle de temps court, ce qui lui permet notamment d'améliorer son endurance, par exemple lors d'une préparation avant une compétition.

Selon des aspects avantageux mais non obligatoires de l'invention, une telle méthode peut incorporer une ou plusieurs des caractéristiques suivantes, prises dans toute combinaison techniquement admissible :

- L'exposant auquel la puissance moyenne est élevée à l'étape d) est choisi de telle façon qu'il augmente le poids de la puissance moyenne dans le calcul de l'énergie, et est de préférence compris entre 0,1 et 4.
- Le nombre de sessions par semaine est calculé sur une période de jeu glissante comprenant un nombre prédéterminé de sessions de jeu.
- Le nombre de sessions de jeu pris en compte par semaine est limité par une valeur maximale.
- La méthode comprend une étape supplémentaire f) consistant à affiner et à ramener la valeur du paramètre calculée à l'étape e) sur une échelle prédéterminée et selon la formule :

$$Px = Px\max \times \left(\frac{P}{P\max}\right)^{j}$$

où Px est la nouvelle valeur du paramètre sur l'échelle prédéterminée, Pxmax est la valeur maximale de l'échelle prédéterminée, Pmax est la valeur maximale du paramètre calculée à l'étape e), et j est un exposant prédéterminé.
- A l'étape f), on fixe x=100 et le paramètre est calculé sous la forme d'un pourcentage.
- A l'étape f), la valeur de l'exposant prédéterminé est comprise entre 0,1 et 1.
- La valeur du paramètre est limitée à une valeur maximale.
- La période de jeu sur laquelle le nombre de sessions de jeu par semaine est calculé comprend un nombre minimal de sessions de jeu.
- La méthode comprend une étape supplémentaire consistant à calculer la moyenne des valeurs du paramètre sur toutes les sessions de jeu prises en compte de la période de jeu.
- La méthode comprend une étape supplémentaire consistant à exporter, vers une interface de calcul dans un appareil de calcul et d'affichage de données, les données mesurées à l'étape a).
- La méthode comprend une étape consistant à limiter l'augmentation du nombre de sessions par semaine entre deux sessions de jeu consécutives à une valeur maximale.L'invention sera mieux comprise et d'autres avantages de celle-ci apparaîtront plus clairement à la lumière de la description qui va suivre, d'un mode de réalisation d'une

méthode de calcul d'un paramètre conforme à son principe, et faite en se référant aux dessins annexés dans lesquels :

- la figure 1 est une vue de face d'une raquette de tennis permettant la mise en oeuvre de la méthode de calcul selon l'invention ; et

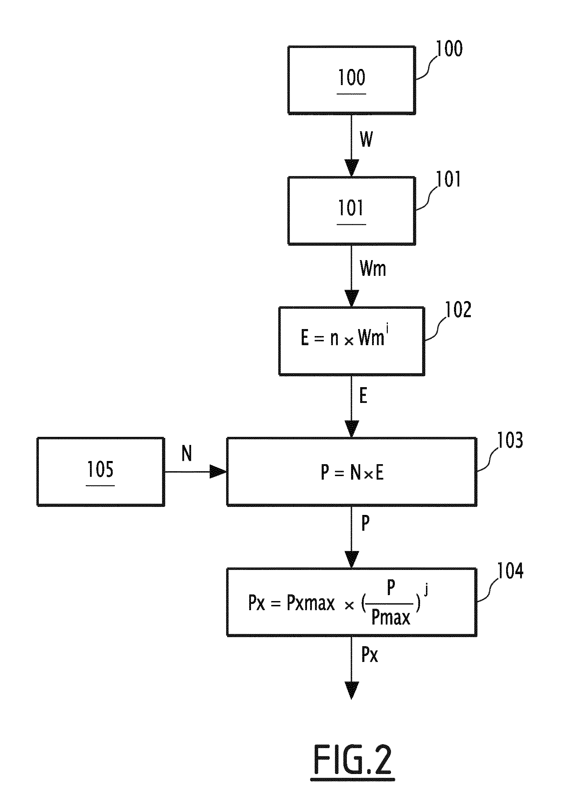

- la figure 2 est un schéma bloc de la méthode selon l'invention.

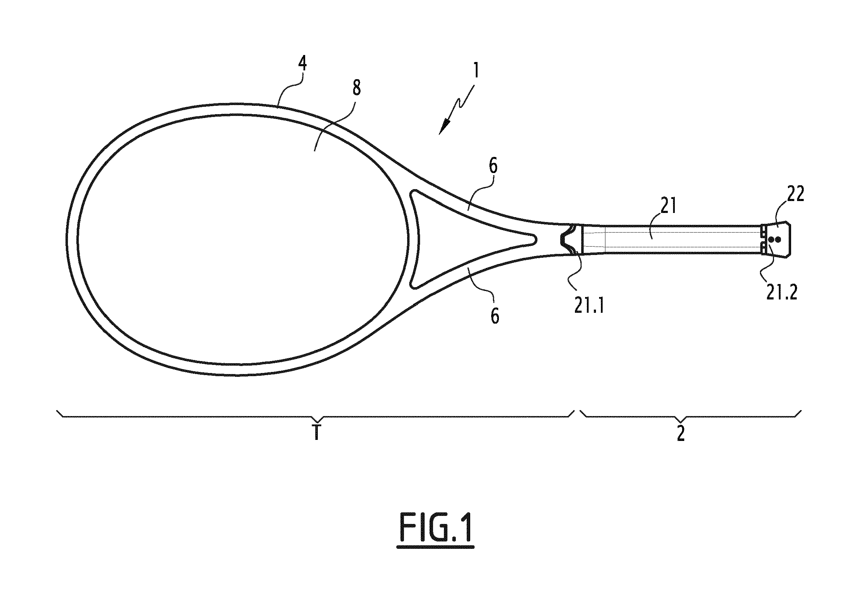

La figure 1 montre une raquette de tennis 1, qui comprend de manière classique un manche 2 raccordé à une tête T incluant un cadre 4 à l'intérieur duquel est tendu un tamis 8, formé de différentes cordes transversales et longitudinales. La tête T comprend également deux branches de liaison 6 qui s'étendent de manière divergente depuis le manche 2 vers le cadre 4.

Le manche 2 comprend un corps 21 globalement tubulaire, réalisé par exemple en carbone. De manière classique, le corps 21 est recouvert d'une épaisseur de mousse entourée par un grip, pour améliorer la prise en main du manche. Le corps 21 du manche 2 comporte une extrémité longitudinale proximale 21.1, située du côté du tamis 8 et raccordée aux branches de liaison 6, et une extrémité longitudinale distale 21.2, libre et opposée à l'extrémité 21.1. L'extrémité 21.2 est recouverte par un talon 22, généralement désigné par le terme « butt cap » en Anglais, qui permet à la main du joueur de ne pas glisser hors du manche 2.

La raquette 1 est équipée d'un système de mesure de données de jeu, non représenté, comprenant des capteurs de mesure aptes à mesurer, lors de la frappe d'une balle avec la raquette 1, l'accélération linéaire et la vitesse angulaire de la raquette 1, selon trois directions perpendiculaires entre elles, fixes par rapport au référentiel terrestre. Par exemple, le système de mesure peut comprendre un accéléromètre tri-axes et un gyromètre tri-axes.

La suite de la description concerne une méthode de calcul d'un paramètre représentatif de l'endurance d'un joueur de tennis, au moyen de la raquette 1.

Lors d'une période de jeu comprenant plusieurs sessions de jeu, les données de jeu sont mesurées par le système de mesure, lorsqu'un joueur réalise des séries de coups (coup droit lifté, service, revers slicé...). Les données recueillies sont de préférence exportées vers une unité de calcul dans un appareil de calcul et d'affichage de données, par exemple un ordinateur ou un téléphone intelligent. Par exemple, l'unité de calcul peut être un microprocesseur.

Dans une première étape 100, la puissance W développée par le joueur pour chaque coup frappé est calculée par l'unité de calcul. La puissance W est ici définie comme une grandeur spécifique calculée à partir du rapport entre la vitesse angulaire et l'accélération linéaire mesurées par les capteurs du système de mesure de la raquette 1. La puissance W est exprimée de préférence par un pourcentage, à partir d'une valeur comprise entre 0 et 1.

Dans une seconde étape 101, la puissance moyenne Wm développée par le joueur pendant la dernière session de jeu est calculée par l'unité de calcul. La puissance moyenne est définie comme la moyenne des puissances W calculées à l'étape 100.

Dans une étape 102, l'énergie E dépensée par le joueur pendant la dernière session de jeu est calculée par l'unité de calcul. L'énergie E est égale au nombre de coups n exécutés par le joueur pendant la session de jeu, multiplié par la puissance moyenne Wm calculée à l'étape 101 élevée à un exposant i, selon la relation $E = n \times Wm^i$, où i est un exposant prédéterminé.

Dans un mode de réalisation préférentiel, l'exposant i est choisi de telle manière qu'il augmente le poids de la puissance moyenne Wm dans le calcul de l'énergie E. L'exposant i est, de préférence, compris entre 0,1 et 4. Dans l'exemple décrit, la puissance moyenne Wm étant une valeur inférieure à 1, l'exposant i peut être choisi entre 0,1 et 1 pour augmenter le poids de la puissance moyenne Wm. A titre d'exemple, i peut être égal à 0,75. La valeur de l'énergie E peut être multipliée par un coefficient correcteur afin de ramener la valeur de l'énergie E sur une échelle prédéterminée.

Dans une étape 103, la valeur du paramètre P est calculée par l'unité de calcul. La valeur P est définie par le produit du nombre N de sessions de jeu réalisées par semaine, au cours de la période de jeu, multiplié par l'énergie calculée à l'étape 102.

Le paramètre P est donc calculé pour chaque session de jeu, en tenant compte des données de jeu de la dernière session de jeu et de la fréquence de jeu sur une période de jeu comprenant la dernière session de jeu.

Le nombre N de sessions de jeu par semaine est calculé sur une période glissante comprenant un nombre prédéterminé de sessions de jeu, par exemple six sessions de jeu. Cela signifie qu'à chaque fois que les données concernant une nouvelle session de jeu sont enregistrées, les données concernant cette nouvelle session remplacent les données de la plus ancienne des six sessions considérées.

Le nombre N de sessions par semaine est calculé, dans une étape 105, par l'unité de calcul en déterminant le nombre de semaines nécessaire à l'obtention de six sessions de jeu, puis en établissant le nombre de sessions de jeu par semaine correspondant.

Si le nombre total de sessions de jeu effectué est insuffisant, le nombre N de sessions par semaine est calculé sur un nombre inférieur de sessions de jeu mais au moins égal à une valeur minimale, par exemple deux sessions de jeu.

Pour éviter que le paramètre d'endurance n'atteigne une valeur incohérente si le joueur multiplie les sessions de jeu, le nombre N de sessions de jeu par semaine est limité à une valeur maximale, par exemple sept sessions de jeu par semaine.

Avantageusement, la valeur du paramètre P est ramenée, dans une étape 104, sur une échelle prédéterminée, à une valeur Px, par exemple un pourcentage P100. La nouvelle valeur Px est néanmoins affinée pour ne pas pénaliser les joueurs qui ne jouent qu'environ une fois par semaine, au moyen de la formule suivante :

$$Px = Px\max \times \left(\frac{P}{P\max}\right)^{j}$$

Où :

- Px est la valeur du paramètre sur une échelle de 0 à une valeur x,
- Pxmax est la valeur maximale de l'échelle choisie et
- Pmax est la valeur maximale du paramètre calculée à l'étape 103.
- j est un exposant prédéterminé.

La valeur de l'exposant j est de préférence comprise entre 0,1 et 1, ce qui permet d'obtenir une valeur d'endurance qui ne pénalise pas trop les joueurs qui jouent un nombre N réduit de sessions par semaines, en définissant une courbe hyperboloïde croissant rapidement pour les valeurs faibles de N.

Dans l'exemple décrit, pour obtenir le pourcentage P100, on fixe x=100 et Pxmax = 100. Dans ce cas, le paramètre P s'écrit donc sous la forme du pourcentage P100 donné par la formule :

$$P100 = 100 \times \left( \frac{P}{P\max} \right)^{j}$$

En variante, la valeur de P peut également être calculée sur une échelle de 0 à 50, en fixant x=50 et en calculant P50, ou une échelle de 0 à 25, en fixant x=25 et en calculant P25.

La valeur maximale Pmax est fixée, par exemple, à 10000, pour éviter des résultats incohérents.

Le paramètre d'endurance P reflète la capacité du joueur à pratiquer des sessions de jeu à haute dépense d'énergie, en mettant une puissance importante dans ses frappes, et à répéter de telles sessions sur une base régulière.

Dans une étape supplémentaire optionnelle, la moyenne des valeurs du paramètre P sur les six sessions de jeu considérées pour le calcul du paramètre P peut être calculée.

Afin d'éviter que la valeur du paramètre P augmente de façon trop brutale si un joueur jouant régulièrement recommence à jouer régulièrement après une longue période d'inactivité, la méthode comprend une étape consistant à limiter l'augmentation du nombre N de session par semaine entre deux sessions de jeu consécutives à une valeur maximale, par exemple 2.

Ainsi, un joueur qui jouait souvent et qui avait, par exemple, un nombre N égal à 7 et qui, pour une raison quelconque, a une période d'inactivité pendant laquelle il ne va pas jouer pendant deux mois, va voir son nombre N et son paramètre d'endurance P chuter. Lorsqu'il va reprendre son rythme de jeu précédant sa période d'inactivité, le calcul du nombre N va toujours prendre en compte la dernière session de jeu de ce joueur avant sa période d'inactivité, le nombre N restant faible jusqu'à qu'il joue six sessions de jeu après sa reprise. A ce moment, la période glissante de six sessions de jeu sur lesquelles le calcul du nombre N ne va plus prendre en compte l'écart de deux mois séparant la dernière session de jeu avant la période d'inactivité et la première session de jeu suivant la période d'inactivité. La limitation de l'augmentation du nombre N entre deux sessions consécutives permet d'éviter que le nombre N n'augmente brusquement à 7, et que le paramètre P augmente de façon trop brutale. Le nombre N va progressivement augmenter pour revenir à sa valeur d'avant l'interruption de deux mois.

## Revendications

1. Méthode de calcul d'un paramètre (P) représentatif de l'endurance d'un joueur de tennis au cours d'une période de jeu comprenant une série de sessions de jeu, **caractérisée en ce qu'**elle comprend au moins des étapes consistant à :

   - a) mesurer, à l'aide d'un système de mesure comprenant des capteurs implantés sur une raquette (1) utilisée par le joueur, l'accélération linéaire et la vitesse angulaire de la raquette (1) lors de chaque coup frappé par le joueur au cours d'une session de jeu ;
   - b) calculer (100), au moyen d'une unité de calcul, la puissance (W) développée par le joueur lors de chaque coup à partir des accélérations linéaires et des vitesses angulaires mesurées à l'étape a) ;
   - c) calculer (101) au moyen de l'unité de calcul, la puissance moyenne (Wm) développée par le joueur pendant la dernière session de jeu, la puissance moyenne étant définie comme la moyenne des puissances (W) développées lors de chaque coup par le joueur calculées à l'étape b) ;
   - d) calculer (102) au moyen de l'unité de calcul l'énergie (E) dépensée par le joueur pendant la dernière session de jeu, l'énergie dépensée étant égale au nombre (n) de coups exécutés par le joueur au cours de la dernière session de jeu multipliée par la puissance moyenne (Wm) calculée à l'étape c) élevée à un exposant (i) prédéterminé ;
   - e) calculer (103) au moyen de l'unité de calcul la valeur du paramètre (P), cette valeur étant définie par le produit du nombre (N) de sessions de jeu réalisées par semaine au cours de la période de jeu, multiplié par l'énergie (E) calculée à l'étape d).

2. Méthode selon la revendication 1, **caractérisée en ce que** l'exposant (i) auquel la puissance moyenne (Wm) est élevée à l'étape d) est choisi de telle manière qu'il augmente le poids de la puissance moyenne (Wm) dans le calcul de l'énergie (E) et est compris de préférence entre 0,1 et 4.

**3.** Méthode selon l'une des revendications précédentes, **caractérisée en ce que** le nombre (N) de sessions par semaine est calculé (105) sur une période de jeu glissante comprenant un nombre prédéterminé de sessions de jeu.

**4.** Méthode selon l'une des revendications précédentes, **caractérisée en ce que** le nombre (N) de sessions de jeu pris en compte par semaine est limité par une valeur maximale.

**5.** Méthode selon l'une des revendications précédentes, **caractérisée en ce qu'**elle comprend une étape supplémentaire f) consistant à affiner et à ramener (104) la valeur du paramètre (P) calculée à l'étape e) sur une échelle prédéterminée et selon la formule :

$$Px = Px\max \times \left( \frac{P}{P\max} \right)^{j}$$

Où :

- Px est la nouvelle valeur du paramètre (P) sur l'échelle prédéterminée,
- Pxmax est la valeur maximale de l'échelle prédéterminée
- Pmax est la valeur maximale du paramètre (P) calculée à l'étape e), et
- j est un exposant prédéterminé.

**6.** Méthode selon la revendication 5, **caractérisée en ce qu'**à l'étape f), on fixe x=100 et **en ce que** le paramètre (P) est calculé sous la forme d'un pourcentage (P100).

**7.** Méthode selon l'une des revendications 5 et 6, **caractérisée en ce qu'**à l'étape f), la valeur de l'exposant (j) prédéterminé est comprise entre 0,1 et 1.

**8.** Méthode selon l'une des revendications précédentes, **caractérisée en ce que** la valeur du paramètre (P) est limitée à une valeur maximale.

**9.** Méthode selon l'une des revendications précédentes, **caractérisée en ce que** la période de jeu sur laquelle le nombre (N) de sessions de jeu par semaine est calculé comprend un nombre minimal de sessions de jeu.

**10.** Méthode selon l'une des revendications précédentes, **caractérisée en ce qu'**elle comprend une étape supplémentaire consistant à calculer la moyenne des valeurs du paramètre (P) sur toutes les sessions de jeu prises en compte de la période de jeu.

**11.** Méthode selon l'une des revendications précédentes, **caractérisée en ce qu'**elle comprend une étape supplémentaire consistant à exporter, vers une interface de calcul dans un appareil de calcul et d'affichage de données, les données mesurées à l'étape a).

**12.** Méthode selon l'une des revendications précédentes, **caractérisée en ce qu'**elle comprend une étape consistant à limiter l'augmentation du nombre (N) de sessions par semaine entre deux sessions de jeu consécutives à une valeur maximale.

FIG.1

EP 2 839 864 A1

$$100$$

$$\downarrow W$$

$$101$$

$$\downarrow Wm$$

$$E = n \times Wm^i \quad 102$$

$$\downarrow E$$

$$105 \quad \xrightarrow{N} \quad P = N \times E \quad 103$$

$$\downarrow P$$

$$Px = Pxmax \times \left(\frac{P}{Pmax}\right)^j \quad 104$$

$$\downarrow Px$$

## FIG.2

Europäisches
Patentamt

European
Patent Office

Office européen
des brevets

**RAPPORT DE RECHERCHE EUROPEENNE**

Numéro de la demande

EP 14 18 1478

## DOCUMENTS CONSIDERES COMME PERTINENTS

| Catégorie | Citation du document avec indication, en cas de besoin, des parties pertinentes | Revendication concernée | CLASSEMENT DE LA DEMANDE (IPC) |
|---|---|---|---|
| X,P | WO 2013/174922 A1 (BABOLAT VS [FR]; MOVEA [FR]) 28 novembre 2013 (2013-11-28) * page 1, lignes 3-19 * * page 6, ligne 32 - page 7, ligne 5 * ----- | 1-11 | INV. A63B69/38 G09B19/00 |
| X | US 2013/053190 A1 (METTLER BERENICE [US]) 28 février 2013 (2013-02-28) * alinéas [0002], [0003], [0018], [0019], [0023], [0024] * * alinéas [0043], [0044], [0061] - [0063], [0120], [0126], [0127] * ----- | 1-11 | |
| X | EP 2 592 612 A2 (DUNLOP SPORTS CO LTD [JP]) 15 mai 2013 (2013-05-15) * abrégé * * alinéa [0069] - alinéa [0075]; figures 1,2 * * alinéas [0132], [0147] * ----- | 1-11 | |
| A | WO 2011/036567 A2 (SCHWENGER RALF [DE]; KOTZE JOHAN [ZA]; LAMMER HERFRIED [AT] HEAD TECHN) 31 mars 2011 (2011-03-31) * alinéa [0035] - alinéa [0040] * * alinéa [0050] - alinéa [0054] * ----- | 1-11 | **DOMAINES TECHNIQUES RECHERCHES (IPC)** A63B G09B G06F |
| A | WO 2012/082782 A1 (NIKE INTERNATIONAL LTD [US]; HOLMES STEVE [US]; WEAST AARON B [US]) 21 juin 2012 (2012-06-21) * abrégé * * alinéas [0027], [0031], [0035], [0038], [0039], [0055], [0056] * ----- | 1-11 | |

Le présent rapport a été établi pour toutes les revendications

| Lieu de la recherche | Date d'achèvement de la recherche | Examinateur |
|---|---|---|
| Berlin | 28 novembre 2014 | Domingo Vecchioni, M |

CATEGORIE DES DOCUMENTS CITES

X : particulièrement pertinent à lui seul
Y : particulièrement pertinent en combinaison avec un
    autre document de la même catégorie
A : arrière-plan technologique
O : divulgation non-écrite
P : document intercalaire

T : théorie ou principe à la base de l'invention
E : document de brevet antérieur, mais publié à la
    date de dépôt ou après cette date
D : cité dans la demande
L : cité pour d'autres raisons

                                                   

& : membre de la même famille, document correspondant

EPO FORM 1503 03.82 (P04C02)

**ANNEXE AU RAPPORT DE RECHERCHE EUROPEENNE
RELATIF A LA DEMANDE DE BREVET EUROPEEN NO.**

EP 14 18 1478

La présente annexe indique les membres de la famille de brevets relatifs aux documents brevets cités dans le rapport de recherche européenne visé ci-dessus.
Lesdits members sont contenus au fichier informatique de l'Office européen des brevets à la date du
Les renseignements fournis sont donnés à titre indicatif et n'engagent pas la responsabilité de l'Office européen des brevets.

28-11-2014

| Document brevet cité au rapport de recherche | Date de publication | Membre(s) de la famille de brevet(s) | Date de publication |
|---|---|---|---|
| WO 2013174922 A1 | 28-11-2013 | FR 2990876 A1<br>WO 2013174922 A1 | 29-11-2013<br>28-11-2013 |
| US 2013053190 A1 | 28-02-2013 | EP 2750773 A1<br>US 2013053190 A1<br>WO 2013032836 A1 | 09-07-2014<br>28-02-2013<br>07-03-2013 |
| EP 2592612 A2 | 15-05-2013 | EP 2592612 A2<br>US 2013127866 A1 | 15-05-2013<br>23-05-2013 |
| WO 2011036567 A2 | 31-03-2011 | CN 102686285 A<br>DE 202010018140 U1<br>DE 202010018141 U1<br>DE 202010018142 U1<br>EP 2480292 A2<br>EP 2752223 A1<br>EP 2752224 A1<br>JP 2013505761 A<br>US 2011183787 A1<br>US 2014141907 A1<br>WO 2011036567 A2 | 19-09-2012<br>13-05-2014<br>13-05-2014<br>13-05-2014<br>01-08-2012<br>09-07-2014<br>09-07-2014<br>21-02-2013<br>28-07-2011<br>22-05-2014<br>31-03-2011 |
| WO 2012082782 A1 | 21-06-2012 | CA 2821274 A1<br>CN 103620607 A<br>EP 2652658 A1<br>JP 2014502527 A<br>KR 20130116898 A<br>WO 2012082782 A1 | 21-06-2012<br>05-03-2014<br>23-10-2013<br>03-02-2014<br>24-10-2013<br>21-06-2012 |

EPO FORM P0460

Pour tout renseignement concernant cette annexe : voir Journal Officiel de l'Office européen des brevets, No.12/82